# EUROPEAN PATENT SPECIFICATION

(11) **EP 4 537 889 B1**
(45) Date of publication and mention of the grant of the patent: **25.11.2026**
(21) Application number: 24199121.5
(22) Date of filing: 25.02.2021
(51) Int. Cl.: A61M 39/10

(54) **FLUID MIXING SET**
FLUIDMISCHSET
SET DE MELANGE DE FLUID

(30) Priority: 28.02.2020 US 202062982995 P
(43) Date of publication of application: 16.04.2025
(62) Document number(s) of the earlier application(s) in accordance with Art. 76 EPC: 21713224.0 / 4 110 452
(73) Proprietor: Bayer Healthcare LLC, Whippany, NJ 07981 (US)
(72) Inventor: Cowan, Kevin, Pennsylvania, 4242 (US); Dedig, James, Pennsylvania, 15220 (US); Spohn, Michael, Pennsylvania, 16034 (US); Haury, John, Pennsylvania, 15143 (US)
(74) Representative: BIP Patents

(56) References cited:
- WO-A1-2014/162337
- GB-A- 1 173 662
- US-A- 3 868 967
- US-A- 5 431 185
- US-A1- 2012 029 479
- US-A1- 2014 261 713
- US-B2- 9 180 260

## Description

### BACKGROUND OF THE DISCLOSURE

### Field of the Disclosure

The present disclosure is related to fluid mixing devices for use with fluid delivery tubing sets configured for use with powered fluid injectors. The present disclosure is also related to fluid delivery tube sets having said fluid mixing devices.

### Description of Related Art

In many medical diagnostic and therapeutic procedures, a medical practitioner, such as a physician or radiologist, injects a patient with one or more fluids using a powered fluid injector system. In recent years, a number of powered fluid injector systems for pressurized injection of fluids have been developed for use in procedures such as angiography (CV), computed tomography (CT), molecular imaging (such as PET imaging), and magnetic resonance imaging (MRI). In these imaging procedures, a first injection fluid, such as a contrast agent, may be used to highlight certain internal organs, portions of the circulatory system, or portions of the body during an imaging process. Meanwhile, a second injection fluid, such as saline or a similar flushing agent, may be used to ensure complete injection of the bolus of the contrast agent and/or adjust the concentration of the contrast agent. In some procedures, it may be desirable to deliver a mixture of the first injection fluid and the second injection fluid.
Fluid mixing devices for hot and cold water are known in the art. For example, US3,868,967 discloses an adapter for mixing fluids comprising a body provided with a primary chamber having an outlet passage, a plurality of inlet passages communicatively connected to said chamber whereby streams of fluid of different character when forced through said inlet passages will be mixed in said chamber prior to flow outwardly through said outlet passage, and means in or adjacent one or more of the aforesaid passages whereby to facilitate mixing of the fluids in the chamber and retard back flow of the mixture into either or both of the inlet passages whenever the pressure varies in the latter.
US2014/0261713 A1 discloses a fluid mixing device in a fluid path set for a first and second fluid such as contrast agent and flushing fluid. The fluid path set includes a first fluid line having a proximal end fluidly connectable to a source of a first fluid and a second fluid line having a proximal end fluidly connectable to a source of a second fluid. A flow mixing device is in fluid communication with distal ends of the first and second fluid lines. The flow mixing device includes a housing, a first fluid port provided for receiving the first fluid, and a second fluid port for receiving the second fluid. A mixing chamber is disposed within the housing and is in fluid communication with the first and second fluid ports. A third fluid port in fluid communication with the mixing chamber for discharging a mixed solution of the first and second fluids. A turbulent flow inducing member is disposed within the mixing chamber for promoting turbulent mixing of the first and second fluids.

When delivering a mixture of the first injection fluid and the second injection fluid, it is desirable for the two fluids to be mixed well before injection into the patient. However, because the first and second injection fluids typically have different physical properties, for example specific gravity and/or viscosity, the two fluids may not be thoroughly mixed prior to entering the patient's vascular system, leading to reduced image quality. Accordingly, there is a need in the art for improved fluid delivery systems that promote mixing of two or more injection fluids prior to injection into the patient.

### SUMMARY OF THE DISCLOSURE

These needs and others may be met by the non-limiting embodiments described herein, which are directed to an improved fluid mixing devices and fluid delivery tube sets including the same. Subject-matter of the present invention is a fluid mixing device according to claim 1.

In some non-limiting embodiments of the present invention, the fluid mixing device further may include at least one of a first check valve in the first fluid inlet, and a second check valve in the second fluid inlet.

In some non-limiting embodiments of the present invention, the mixing chamber further may include a first inlet, wherein the first inlet of the mixing chamber is distal to the third redirecting surface. The first redirecting surface may be positioned distal to the first fluid inlet and at least partially faces the first inlet to the mixing chamber. The mixing chamber further may include a second inlet, wherein the second inlet of the mixing chamber is -distal to the third redirecting surface. The second redirecting surface may be positioned distal to the second fluid inlet and at least partially faces the second inlet to the mixing chamber.

In some non-limiting embodiments of the present invention, at least one of the first redirecting surface and the second redirecting surface may be substantially concave and have a radius of curvature greater than or equal to 90°. At least one of the first redirecting surface and the second redirecting surface may be substantially concave and have a radius of curvature greater than or equal to 150⁰. The third redirecting surface may have a substantially concave-shaped surface facing the outlet port. The concave-shaped surface may have a radius of curvature of greater than or equal to 90°. The concave-shaped surface may have a radius of curvature of greater than or equal to 150⁰.

In some non-limiting embodiments of the present invention, the first check valve may have a first end in engagement with a first inlet port on the first fluid inlet and a second end in engagement with a first stop element proximal to the first redirecting surface. The second check valve may have a first end in engagement with a second inlet port on the second fluid inlet and a second end in engagement with a second stop element proximal to the second redirecting surface. The first check valve and the second check valve may be reversibly compressible between the first end and the second end in response to first fluid pressure of the first injection fluid flowing through the first inlet port and a second fluid pressure of the second injection fluid flowing through the second fluid port, respectively. The first stop element and the second stop element may have a pointed proximal end.

In some non-limiting embodiments of the present invention, the outlet port may have an axis parallel to an axis of the first fluid inlet and an axis of the second fluid inlet. The axis of the outlet port may extend between the axis of the first fluid inlet and the axis of the second fluid inlet.

In some non-limiting embodiments of the present invention, the outlet port further may include a pressure isolation valve integrated therewith.

The pressure isolation valve may have a first lumen in fluid communication with the outlet port, a second lumen configured for connecting to a pressure transducer, and a valve member between the first lumen and the second lumen, wherein the valve member is configured for isolating the second lumen from the outlet port during a fluid injection procedure.

In some non-limiting embodiments of the present invention, a fluid delivery tube set for delivering fluid from a fluid injector to a patient may include: a first inlet tube configured to deliver a first injection fluid; a second inlet tube configured to deliver a second injection fluid; an outlet tube configured to deliver a mixture of the first injection fluid and the second injection fluid to a patient; and the fluid mixing device of claim 1 and the claims dependent thereon.
injection fluid in a first different direction from the first direction to enter the mixing chamber along the first different direction, and the second redirecting surface may be configured to redirect the second injection fluid in a second different direction from the second direction to enter the mixing chamber along the second different direction. The first different direction and the second different direction may be selected so that the first injection fluid and the second injection fluid contact the third redirecting surface of the mixing chamber to turbulently mix the first injection fluid and the second injection fluid in the mixing chamber. A mixture of the first injection fluid and the second injection fluid may exit the fluid mixing device through the outlet port.

In some non-limiting embodiments of the present disclosure, a method for turbulently mixing a first injection fluid and a second injection fluid to form a substantially homogeneous mixture of the first injection fluid and the second injection fluid may include contacting a fluid flow of the first injection fluid with a first concave redirecting surface associated with a first fluid inlet. The method further may include redirecting the fluid flow of the first injection fluid to a first different direction, wherein the first different direction flows at an angle ranging from 90-175° from a fluid flow direction of the first injection fluid and towards a third concave redirecting surface in a mixing chamber. The method further may include contacting a fluid flow of the second injection fluid with a second concave redirecting surface associated with a second fluid inlet. The method further may include redirecting the fluid flow of the second injection fluid to a second different direction, wherein the second different direction flows at an angle ranging from 90-175°from a fluid flow direction of the second injection fluid and towards the third concave redirecting surface in the mixing chamber. The method further may include turbulently mixing the first injection fluid and the second injection fluid in the mixing chamber upon contact of the first injection fluid and the second injection fluid with the third concave redirecting surface to form a mixture of the first injection fluid and the second injection fluid; and redirecting the mixture of the first injection fluid and the second injection fluid through an outlet port of the mixing chamber.

Further details and advantages of the various embodiments described in detail herein will become clear upon reviewing the following detailed description of the various examples in conjunction with the accompanying drawing figures.

### BRIEF DESCRIPTION OF THE DRAWINGS

**FIG. 1** is a perspective view of a fluid injector system according to some embodiments of the present disclosure;
**FIG. 2** is a perspective view of a portion of a fluid delivery tube set that may be used with the fluid injector system of **FIG. 1****;**
**FIG. 3** is a profile view of the fluid mixing device for the fluid delivery tube set of **FIG. 2****;**
**FIG. 4** is a plan view of a distal end of the fluid mixing device of **FIG. 3****;**
**FIG.** 5 is a plan view of a proximal end of the fluid mixing device of **FIG. 3****;**
**FIG. 6** is a cross-sectional view of the fluid mixing device of **FIGS. 3-5****,** taken along line A - A in **FIG. 4****;**
**FIG. 7** is a cross-sectional view of a fluid mixing device according to another embodiment of the present disclosure;
**FIG. 8** is a cross-sectional view of a fluid mixing device according to another embodiment of the present disclosure;
**FIG. 9** is a cross-sectional view of a fluid mixing device according to another embodiment of the present disclosure;
**FIG. 10** is a top view of a fluid mixing device according to an example not forming part of the invention;
**FIG. 11** is a side view of the fluid mixing device shown in **FIG. 10****;**
**FIG. 12** is a cross-sectional view of the fluid mixing device of **FIGS. 10-11****,** taken along line B - B in **FIG. 11****;**
**FIG. 13** is a perspective view of a fluid mixing device according to an example not forming part of the invention;
**FIG. 14** is a side view of the fluid mixing device shown in **FIG. 13****;**
**FIG. 15** is a cross-sectional view of the fluid mixing device of **FIGS. 13-14****,** taken along line C - C in **FIG. 14****;**
**FIG. 16** is a top view of a fluid mixing device according to an example not forming part of the invention;
**FIG. 17** is a top view of the fluid mixing device shown in **FIG. 16****;**
**FIG. 18** is a cross-sectional view of the fluid mixing device of **FIGS. 16-17****,** taken along line D - D in **FIG. 17****;**
**FIGS. 19-21** are a cross-sectional views of fluid mixing devices according to further examples not forming part of the invention;
**FIG. 22** is a perspective view of a fluid mixing device according to another embodiment;
**FIG. 23** is an exploded view of a fluid mixing device shown in **FIG. 22****;**
**FIG. 24A** is a cross-sectional view of the fluid mixing device of **FIGS. 22-23****,** taken along line E - E in **FIG. 22****,** with a check valve shown in a closed position;
**FIG. 24B** is a cross-sectional view of the fluid mixing device of **FIGS. 22-23****,** taken along line E - E in **FIG. 22****,** with a check valve shown in an open position;
**FIG. 25** is a cross-sectional view of the fluid mixing device of **FIGS. 22-23****,** taken along line F - F in **FIG. 22****;**
**FIG. 26** is a cross-sectional view of a fluid inlet of the fluid mixing device shown in **FIG. 25** taken along line G - G in **FIG. 25****;**
**FIG. 27** is a cross-sectional view of the fluid inlet of the fluid mixing device shown in **FIG. 25** taken along line H - H in **FIG. 25****;**
**FIG. 28** is a perspective view of a fluid mixing device coupled with a pressure isolation valve according to another embodiment;
**FIG. 29** is an exploded view of the fluid mixing device shown in **FIG. 28****;** and
**FIG. 30** is a cross-sectional view of the fluid mixing device of **FIGS. 28-29****,** taken along line I - I in **FIG. 28****.**

### DETAILED DESCRIPTION OF THE DISCLOSURE

For purposes of the description hereinafter, the terms "upper", "lower", "right", "left", "vertical", "horizontal", "top", "bottom", "lateral", "longitudinal", and derivatives thereof shall relate to the disclosure as it is oriented in the drawing figures.

Spatial or directional terms, such as "left", "right", "inner", "outer", "above", "below", and the like, are not to be considered as limiting as the disclosure can assume various alternative orientations.

All numbers used in the specification and claims are to be understood as being modified in all instances by the term "about". The terms "approximately", "about", and "substantially" mean a range of plus or minus ten percent of the stated value.

Unless otherwise indicated, all ranges or ratios disclosed herein are to be understood to encompass the beginning and ending values and any and all subranges or sub-ratios subsumed therein. For example, a stated range or ratio of "1 to 10" should be considered to include any and all subranges or subratios between (and inclusive of) the minimum value of 1 and the maximum value of 10; that is, all subranges or subratios beginning with a minimum value of 1 or more and ending with a maximum value of 10 or less. The ranges and/or ratios disclosed herein represent the average values over the specified range and/or ratio.

The terms "first", "second", and the like are not intended to refer to any particular order or chronology, but refer to different conditions, properties, or elements.

The term "at least" is synonymous with "greater than or equal to".

As used herein, the term "at least one of" is synonymous with "one or more of". For example, the phrase "at least one of A, B, and C" means any one of A, B, and C, or any combination of any two or more of A, B, and C. For example, "at least one of A, B, and C" includes one or more of A alone; or one or more B alone; or one or more of C alone; or one or more of A and one or more of B; or one or more of A and one or more of C; or one or more of B and one or more of C; or one or more of all of A, B, and C. Similarly, as used herein, the term "at least two of" is synonymous with "two or more of". For example, the phrase "at least two of D, E, and F" means any combination of any two or more of D, E, and F. For example, "at least two of D, E, and F" includes one or more of D and one or more of E; or one or more of D and one or more of F; or one or more of E and one or more of F; or one or more of all of D, E, and F.

The word "comprising" and "comprises", and the like, does not exclude the presence of elements or steps other than those listed in any claim or the specification as a whole. In the present specification, "comprises" means "includes" and "comprising" means "including".

As used herein, the terms "parallel" or "substantially parallel" mean a relative angle as between two objects (if extended to theoretical intersection), such as elongated objects and including reference lines, that is from 0° to 5°, or from 0° to 3°, or from 0° to 2°, or from 0° to 1°, or from 0° to 0.5°, or from 0° to 0.25°, or from 0° to 0.1°, inclusive of the recited values.

As used herein, the terms "perpendicular", "transverse", "substantially perpendicular", or "substantially transverse" mean a relative angle as between two objects at their real or theoretical intersection is from 85° to 90°, or from 87° to 90°, or from 88° to 90°, or from 89° to 90°, or from 89.5° to 90°, or from 89.75° to 90°, or from 89.9° to 90°, inclusive of the recited values.

It is also to be understood that the specific devices and processes illustrated in the attached drawings, and described in the following specification, are simply exemplary examples of the disclosure. Hence, specific dimensions and other physical characteristics related to the examples disclosed herein are not to be considered as limiting.

When used in relation to a component of a fluid injector system such as a fluid reservoir, a syringe, or a fluid line, the term "distal" refers to a portion of said component nearest to a patient. When used in relation to a component of a fluid injector system such as a fluid reservoir, a syringe, or a fluid line, the term "proximal" refers to a portion of said component nearest to the injector of the fluid injector system (i.e., the portion of said component farthest from the patient). When used in relation to a component of a fluid injector system such as a fluid reservoir, a syringe, or a fluid line, the term "upstream" refers to a direction away from the patient and towards the injector of the fluid injector system. For example, if a first component is referred to as being "upstream" of a second component, the first component is located nearer to the injector along the fluid path than the second component is to the injector. When used in relation to a component of a fluid injector system such as a fluid reservoir, a syringe, or a fluid line, the term "downstream" refers to a direction towards the patient and away from the injector of the fluid injector system. For example, if a first component is referred to as being "downstream" of a second component, the first component is located nearer to the patient along the fluid path than the second component is to the patient.

Although the present disclosure is described primarily in reference to the MEDRAD^{®} Stellant CT Injection System, it will be apparent to persons of ordinary skill in the art that the present disclosure can be applied to a variety of injection systems inclusive of their associated disposables (e.g., syringes, tubing, etc.), such as those designed for CT, CV, MR, PET, ultrasound, and other medical injectors configured to inject two or more medical fluids.. In certain embodiments, the fluid mixing device may be suited for use with tubing associated with an angiography injector. Examples of such injection systems include the MEDRAD^{®} Salient CT Injection System, MEDRAD^{®} Stellant FLEX CT Injection System, MEDRAD^{®} Centargo CT Injection System, MEDRAD^{®} MRXperion MR Injection System, MEDRAD^{®} Avanta Injection System, and MEDRAD^{®} Mark 7 Arterion Injection System offered by Bayer HealthCare LLC, Indianola, PA.

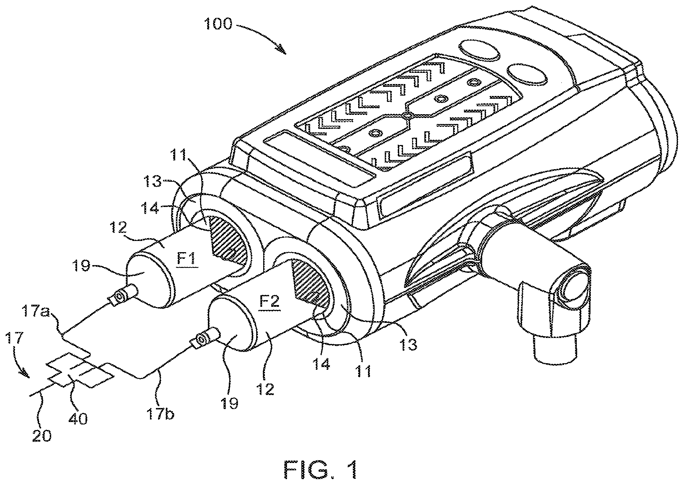

Referring now to **FIG. 1****,** a non-limiting example of a fluid injector system **100** in accordance with the present disclosure includes at least one fluid reservoir, such as at least one syringe **12** having a reciprocally-movable plunger **14,** at least one piston connectable to the plunger **14,** and a fluid control module (not pictured). The fluid injector system **100** may be configured as a computed tomography (CT) contrast injector system, a magnetic resonance imaging (MRI) contrast injector system, or an angiographic (CV) contrast injector system. The at least one syringe **12** is generally adapted to interface with at least one component of the system, such as a syringe port **13.** The fluid injector system **100** is generally configured to deliver at least one fluid **F** from the at least one syringe **12** to a patient during an injection procedure. The fluid injector system **100** is configured to releasably receive the at least one syringe **12,** which is to be filled with at least one fluid **F,** such as a contrast media, saline solution, Ringer's lactate, or any desired medical fluid. The system may be a multi-syringe injector, wherein several syringes may be oriented side-by-side or in another spatial relationship and are separately actuated by respective pistons associated with the injector. The at least one syringe **12** may be oriented in any manner such as upright, downright, or positioned at any degree angle.

With continued reference to **FIG. 1****,** the injector system **100** may be a dual syringe fluid injector system used during a medical procedure to inject the at least two injection fluids **F1** and **F2** into the vasculature system of a patient by driving plungers **14** of respective syringes **12** with a drive member, such as a piston (not shown). Alternatively, one or both of the syringes of the dual head fluid injector system may be replaced with a pump, such as a peristaltic pump, without deviating from the scope of the present disclosure. The first and second injection fluids **F1** and **F2** may be a suitable contrast imaging agent and a flushing fluid, respectively. The piston may be configured to engage the plunger **14.** Upon engagement, the at least one piston may move the plunger **14** toward the distal end **19** of the at least one syringe **12,** for example during a fluid delivery operation, as well as retracting the plunger **14** toward the proximal end **11** of the at least one syringe **12,** for example during a filling operation to fill the syringe **12.**

According to various embodiments, a tubing set **17** (e.g., first and second fluid conduits **17a** and **17b** configured for connecting to respective first and second syringes **12** and common administration line **20**) may be in fluid communication with an outlet port of each syringe **12** to place each syringe in fluid communication with a catheter or other fluid delivery device for delivering the fluid **F** from each syringe **12** to the a vascular access site. The first and second fluid conduits **17a** and **17b** may be connected to the common administration line **20** by a fluid mixing device **40** according to various embodiments of the present disclosure. The fluid injector system **100** shown in **FIG. 1** is an open system do to the lack of valves configured of isolating the syringes **12** from one another and from at least a portion of the tubing set **17.** However, it is to be understood that valves may be added distally of the syringes **12** to convert the fluid injector system **100** of **FIG. 1** to a closed system.

For accurate and efficient administration of volumes of contrast agent during an imaging procedure, many injection protocols require a dual flow administration, i.e., where a mixture of both contrast agent and saline are administered concurrently to the patient. However, because the contrast and the flushing fluid (saline) typically have different physical properties, for example specific gravity, viscosity, and/or surface tension properties, the two solutions may not be thoroughly mixed prior to entering the patient's vascular system leading to reduced image quality. For example, in certain cases where inefficient mixing has occurred, laminar flow of the less viscous faster flowing fluid may occur past the more viscous, slower flowing fluid. While Y-connectors and T-connectors for connecting two fluid conduits to a common administration line are known, conventional Y-connectors and T-connectors may not provide sufficient mixing of the two fluids. Turbulent mixing may improve the efficiency of mixing between the viscous contrast agent and less viscous saline. Examples of connectors having turbulent mixing chambers are described in U.S. Patent No. 9,555,379. The present disclosure describes new fluid mixing devices that provide improved mixing of viscous and less viscous fluids for contrast enhanced imaging procedures.

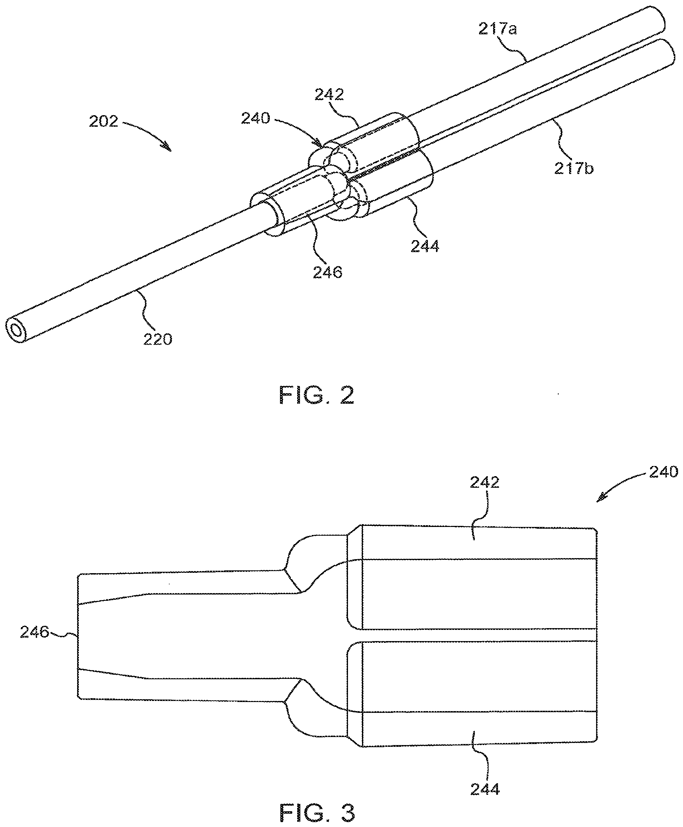

**FIG. 2** is a perspective view of a portion of a fluid delivery tube set **202** that may be used with a dual-head injector, such as the fluid injector system **100** of **FIG. 1** in place of the tubing set **17,** according to some non-limiting embodiments of the present disclosure. As shown, the fluid delivery tube set **202** includes a first inlet line **217a,** a second inlet line **217b,** an outlet line **220,** and a fluid mixing device **240.** The first and second inlet lines **217a** and **217b** are configured to deliver first and second injection fluids, respectively, to the fluid mixing device **240.** In one example embodiment, the first and second injection fluids are a contrast media solution and a saline solution, respectively. Furthermore, the outlet line **220** is configured to deliver a mixture of the first and second injection fluids from the fluid mixing device **240** to a patient or other downstream fluid path component (e.g., a prime tube).

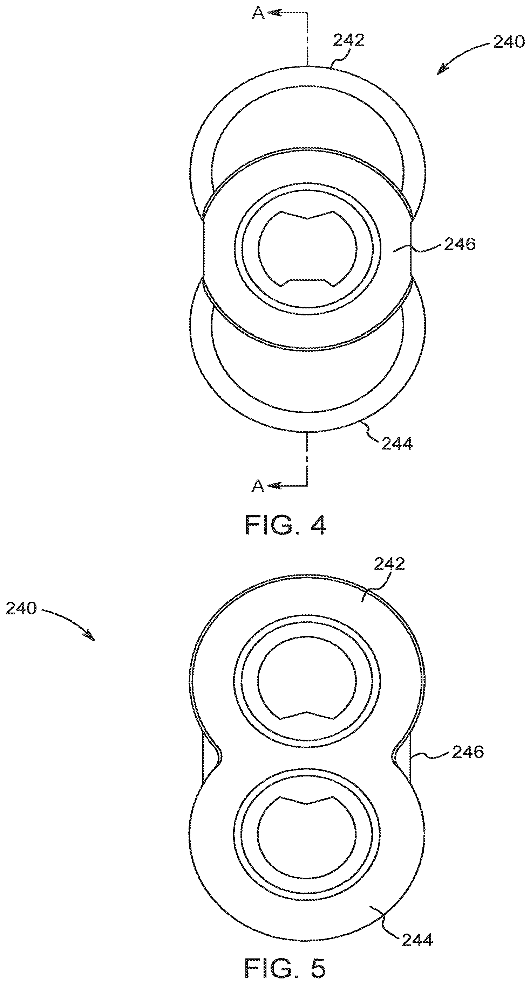

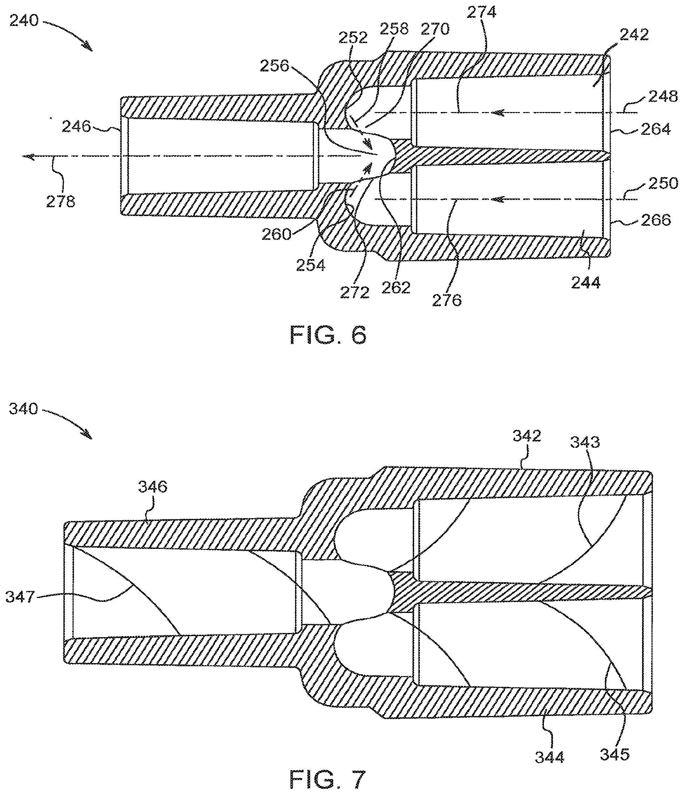

As will be appreciated herein, the fluid mixing device **240** is configured to mix the first and second injection fluids. **FIGS. 3****,** **4****,** **5****,** and **6** show top, left, right, and cross-section views, respectively, of the fluid mixing device **240.** As shown in **FIG. 6****,** the fluid mixing device **240** has a body defining first and second fluid inlets **242** and **244,** each of which is configured to conduct a corresponding one of the first and second injection fluids in a corresponding first and second direction **248** and **250.** As shown, the second direction **250** is along a different axis **276** from the first direction **248.** In certain embodiments, the axis of the first direction **248** and the axis of the second direction **250** may be substantially parallel. In other embodiments, the axis of the first direction **248** may be angled at an acute or an obtuse angle relative to the second direction **250.**

With continuing reference to **FIG. 6****,** the first and second fluid inlets **242** and **244** have corresponding first and second redirecting surfaces **252** and **254..** In certain embodiments, one or both of the first and second redirecting surfaces **252** and **254** are concave-shaped facing the first and second fluid inlets **242** and **244,** respectively, to redirect the flow of fluid. Moreover, the fluid mixing device **240** further has a mixing chamber **256** in fluid communication with the first and second fluid inlets **242** and **244** through first and second mixing chamber inlets **270** and **272,** and an outlet port **246** in fluid communication with the mixing chamber **256.** The mixing chamber **256** is configured to turbulently mix the redirected first and second injection fluids together, for example by turbulently mixing with impact against a third redirecting surface **262** in mixing chamber **256.**

More specifically, the first and second redirecting surfaces **252** and **254** are configured to redirect a first fluid and a second fluid entering the first and second fluid inlets **242** and **244,** respectively, into the mixing chamber **256** through first and second mixing chamber inlets **270** and **272,** where the first and second injection fluids can then be turbulently mixed. Prior to entering the mixing chamber **256,** the first and second injection fluids independently flow through the first and second fluid inlets **242, 244,** respectively. As the first and second fluids flow through the first and second fluid inlets **242, 244,** respectively, the first and second fluids contact the respective first and second redirecting surfaces **252, 254** at distal ends of the first and second fluid inlets **242, 244,** respectively. The first and second redirecting surfaces **252** and **254** are configured to redirect the first and second injection fluids in a corresponding first and second different direction **258** and **260** that is different than the corresponding first and second directions **248** and **250.** Due to this deflection, the first and second injection fluids enter the mixing chamber **256** through first and second mixing chamber inlets **270** and **272** along the corresponding first and second different directions **258** and **260** where the two fluids come into turbulent contact with one another. The first and second different directions **258** and **260** are selected so that the first and second injection fluids contact a third redirecting surface **262** at a proximal end of the mixing chamber **256** to turbulently mix the first and second injections fluids together in the mixing chamber **256.** In some embodiments, the third redirecting surface **262** may have a concave-shaped end facing the outlet port **246.**

After mixing, the mixture of the first and second injection fluids exits the fluid mixing device **240** via the outlet port **246** at a distal end of the fluid mixing device **240** in a direction along a third axis **278.** In some embodiments, the third axis **278** may be parallel with one or both of the first and second axes **274, 276.** In other embodiments, the third axis **278** may be arranged at an acute or obtuse angle relative to both of the first and second axes **274, 276.**

With continued reference to **FIG. 6****,** the first and second fluid inlets **242** and **244** each have corresponding first and second inlet ports **264** and **266,** configured to respectively attach to a first fluid tubing and a second fluid tubing (shown in **FIG. 2**). In some embodiments, the first fluid tubing and the second fluid tubing may be removably or non-removably connectable to the first and second inlet ports **264, 266.** In embodiments where the first fluid tubing and the second fluid tubing are non-removably connectable to the first and second inlet ports **264, 266,** the first fluid tubing and the second fluid tubing may be connected to the first and second inlet ports **264, 266** by solvent bonding, laser welding, or other attachment means.

As shown in **FIG. 6****,** the first and second redirecting surfaces **252** and **254** are positioned distally relative to the first and second inlet ports **264** and **266,** respectively, and the third redirecting surface **262** is positioned proximally relative to the outlet port **246,** and the first and second redirecting surfaces **252** and **254.** In one example embodiment, the first and second redirecting surfaces **252** and **254** are positioned closer to the outlet port **246** compared to the position of the than the third redirecting surface **262** and the outlet port **246.** Furthermore, the first and second redirecting surfaces **252** and **254** may be formed at a distal end of the corresponding first and second fluid inlets **242** and **244,** and each of the first and second redirecting surfaces **252** and **254** at least partially face the corresponding first and second mixing chamber inlets **270** and **272** to the mixing chamber **256,** respectively.

With continued reference to **FIG. 6****,** at least one of the first and second redirecting surfaces **252** and **254** may have a concave surface. Concave surface configuration may improve the redirecting nature of the surface with turbulent flow while eliminating corners in which air bubbles may collect or be temporarily suspended during a priming operation. In some embodiments, each of the first and second redirecting surfaces **252** and **254** may have a radius of curvature greater than or equal to 90°, and in other embodiments being greater than or equal to 150°. For example, in particular embodiments, each of the first and second redirecting surfaces **252** and **254** may have a radius of curvature of from 80° to 160°. In some embodiments, each of the first and second redirecting surfaces **252** and **254** may have a radius of curvature between 90° and 180°. Accordingly, the injection fluid from each of the inlet lines **217a** and **217b** contacts the radiused redirecting surfaces **252** and **254,** which causes the first and second injection fluids to change the flow direction. In some embodiments, the radiused redirecting surfaces **252** and **254** may change the flow direction of the first and second injection fluids, respectively, by an angle ranging from 90° to 150° toward different directions **258** and **260** and into the mixing chamber **256.** As such, the fluids double back and interact with each other, e.g., turbulently mix, in the mixing chamber **256** in combination with further redirection by the third redirecting surface **262.** After the fluids mix to a homogenous solution, the mixture of fluids is redirected again by the radius of the third redirecting surface **262** along a flow direction of the third axis **278** causing the mixture of the first and second injection fluid to flow down the single outlet line **220.** In some embodiments, the third redirecting surface **262** may have a radius of curvature greater than or equal to 90°, more preferably being greater than or equal to 150°. In some embodiments, the third redirecting surface **262** may have a radius of curvature between 90° and 180°. While known mixing devices (not shown) include some swirling of the injection fluids, various conventional mixing devices may still suffer from a density separation, e.g., higher density fluid spinning to the outside of the lower density fluid, which prevents thorough mixing of the first and second fluids. The fluid mixing device **240,** by way of contrast, produces a substantially homogeneous mixture of the first and second injection fluids during the turbulent mixing process.

According to various embodiments, the first and second redirecting surfaces **252** and **254** may include concave-shaped redirecting surfaces that face directions of flow in the first fluid inlet **242** and the second fluid inlet **244,** respectively. Additionally, as shown in **FIG. 6****,** the first fluid inlet **242,** the second fluid inlet **244,** and the outlet port **246** all have corresponding axes **274, 276,** and **278.** In some embodiments, the third axis **278** of the outlet port **246** may be positioned between the first and second axes **274** and **276** of the first and second fluid inlets **242** and **244,** respectively. In other embodiments, the third axis **278** of the outlet port **246** may be positioned above or below the first and second axes **274** and **276** of the first and second fluid inlets **242** and **244,** respectively. In other embodiments, the third axis **278** of the outlet port **246** may be coaxial with one of the first and second axes **274** and **276** of the first and second fluid inlets **242** and **244.** In other embodiments, the first and second different direction **258** and **260** of the fluids entering the mixing chamber **256** may be angled toward each other with a 0 degree to 90 degree angle so that the first and second fluids directly impact each other and turbulently mix.

In operation, the first injection fluid enters the first fluid inlet **242** and the second injection fluid enters the second fluid inlet **244,** each from a corresponding one of the first and second inlet lines **217a** and **217b** (shown in **FIG. 2**). The first and second injection fluids then pass through the respective first and second fluid inlets **242** and **244** until they reach the first and second redirecting surfaces **252** and **254.** When the first injection fluid engages the first redirecting surface **252,** the first fluid is redirected in the direction **258** into the mixing chamber **256.** Similarly, when the second injection fluid engages the second redirecting surface **254** through first mixing chamber inlet **270,** the second fluid is redirected in the direction **260** into the mixing chamber **256.** At this point, the first and second injection fluids, by having been redirected into the mixing chamber **256** through the second mixing chamber inlet **272,** are turbulently mixed together by the flow of the first and second fluids impacting each other and the third redirecting surface **262** in the mixing chamber **256.** The mixture of the first and second injection fluids is simultaneously engaged with the third redirecting surface **262,** upon which time it is redirected through the outlet port **246** and into the outlet line **220,** in order to be delivered to the patient or other downstream fluid path component. According to various embodiments, the first and second fluids may be at least partially redirected to flow in opposite directions, such as one flowing in a clockwise direction and the other flowing in a counterclockwise direction in the mixing chamber **256** such that the flow of the first and second fluids engage and impact each other head on to create turbulent mixing. For example, the change of inertia associated with the impact of one fluid flowing in a clockwise flow direction and the other fluid flowing in a counterclockwise flow direction results in a turbulently mixed solution of the first and second fluid as the two fluids interact within mixing chamber **256.** Depending on the mixing ratio and flow rates of the first and second injection fluids, the first and second injection fluids may mix solely in the mixing chamber **256,** or in the mixing chamber **256** and in the area of at least one of first redirecting surface **252** and second redirecting surface **254.**

**FIG. 7** is a section view of another embodiment of the fluid mixing device **340,** according to another example of the present disclosure where at least one of the first fluid inlet **342,** the second fluid inlet **344,** and the outlet port **346** include a helical "rifling" pattern on an inner surface to further direct and rotate the respective fluid flow in the inlet and/or outlet and increase turbulent mixing of the first and second fluids. The pattern may include one or more at least partially helical protrusions or indentations recessed into the inner surface or protruding from the inner surface of at least one of the first fluid inlet **342,** the second fluid inlet **344,** and the outlet port **346.** The pattern imparts a rotation of the flow of the fluid within the corresponding fluid path. In the example of **FIG. 7****,** the first fluid inlet **342,** the second fluid inlet **344,** and the outlet port **346** each have an at least partially helical-shaped portion **343, 345,** and **347** for generating a corresponding fluid vortex for at least one of the first injection fluid, the second injection fluid, and the mixture of the first and second injection fluids, respectively, as the respective fluids flow through the channels. The helical-shaped portion in one of the inlets or outlet may have directionality (clockwise or counterclockwise) in the same or different direction and may have different dimensions or pitch as the helical-shaped portion in the other portions of the mixing device **340.** Although the first and second fluid inlets **342** and **344** and the outlet port **346** each have helical-shaped portions **343, 345,** and **347,** it will be appreciated that any number of the aforementioned regions may be provided with a helical-shaped portion, without departing from the scope of the disclosed concept. By having the helical-shaped portions **343, 345,** and **347,** mixing may advantageously be further improved. It will be appreciated that the fluid mixing device **340** otherwise functions the same as the fluid mixing device **240** discussed above.

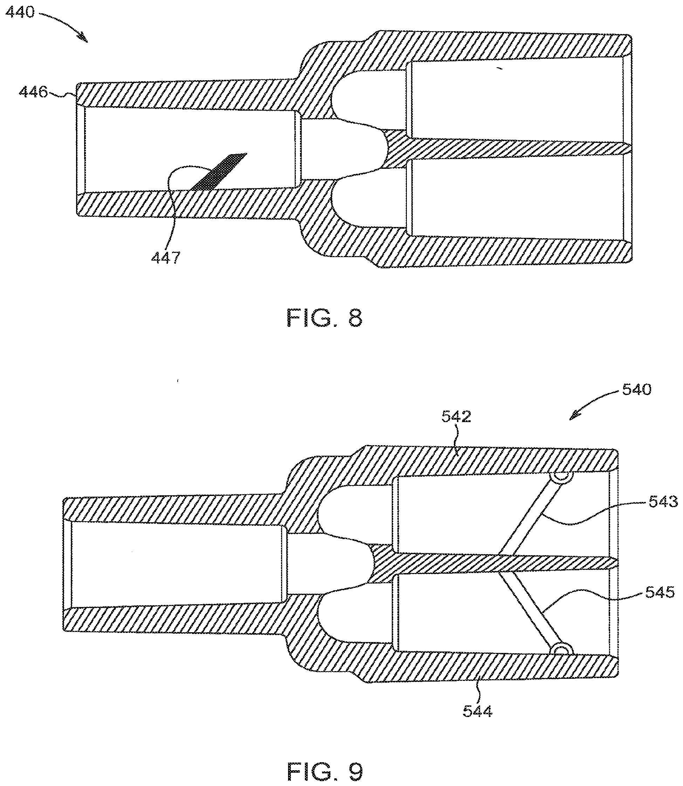

In another embodiment of a fluid mixing device **440** of the present disclosure, as shown in **FIG. 8****,** the outlet port **446** of the fluid mixing device **440** may have one or more baffle members or mixing members **447** located on an interior thereof. The baffle member **447** may advantageously further improve mixing of the first and second injection fluids. It will be appreciated that the fluid mixing device **440** otherwise functions the same as the fluid mixing device **240,** discussed above. In other embodiments, the fluid mixing device may include one or more baffle member or mixing member in one or both of the first and second fluid inlets.

**FIG. 9** shows yet a further example of a fluid mixing device **540,** in accordance with another embodiment of the present disclosure. As shown, the fluid mixing device **540** may include a first valve **543** in the first fluid inlet **542** configured to prevent backflow of the second injection fluid into the first fluid inlet **542** and fluid line **217a.** Furthermore, the fluid mixing device **540** may include a second valve **545** in the second fluid inlet **544** configured to prevent backflow of the first injection fluid into the second fluid inlet **544** and fluid line **217b.** Under the injection pressures typical of a fluid injection procedure, when the pressure of one fluid in the upstream fluid path and fluid inlet is greater than the pressure of the other fluid in the upstream other fluid path and other fluid inlet, backflow of the fluid under greater pressure into the lower pressure fluid path may result in undesired mixing of the fluids in the upstream fluid path or other upstream components of the fluid injection system. This may lead to inaccurate dosing of contrast agent due to the undesired mixing of the two fluid prior to the controlled mixing in the fluid mixing device and may lead to decreased image quality and exposure of the patient to unnecessary excess contrast agent. Otherwise, the fluid mixing device **540** functions the same as the fluid mixing device **240.**

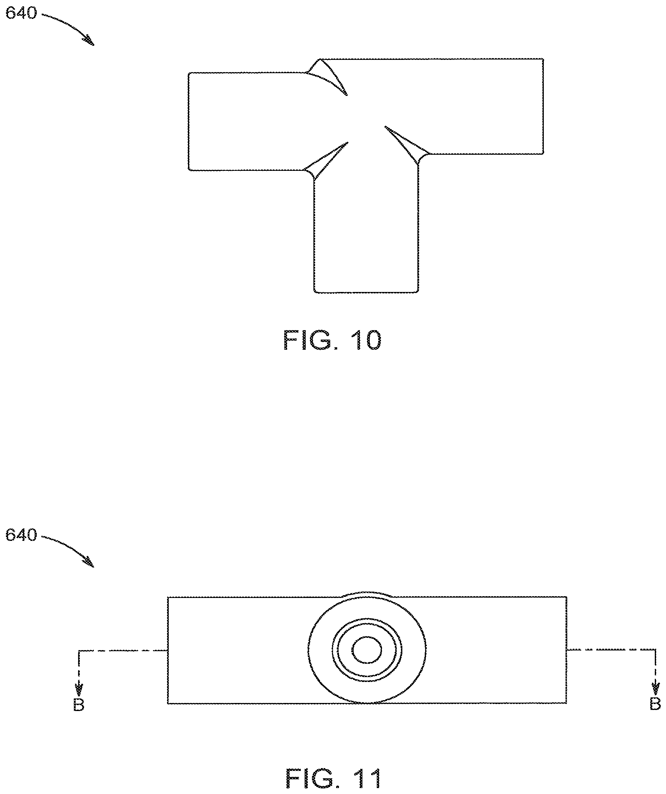

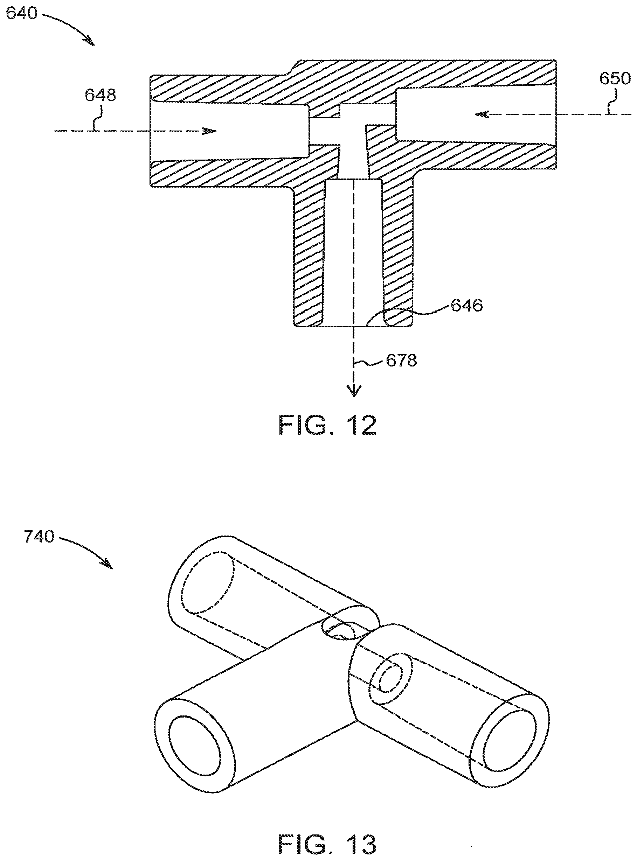

In an example not forming part of the invention of a fluid mixing device **640** of the present disclosure, as shown in **FIGS. 10-12****,** the first direction **648** (**FIG. 12**) is parallel to, in the opposite direction from, and offset from the second direction **650** (**FIG. 12**). Furthermore, as shown, the outlet port **646** of the fluid mixing device **640** has an axis **678** generally perpendicular to the first and second directions **648** and **650.** Accordingly, the fluid mixing device **640** provides indirect instead of head-on mixing of the two fluids. For example, the first direction **648** and the second direction **646** facilitate a direct collision of stream lines of one-half the diameter of the tubing cross-section and indirect mixing of the other one-half of the stream lines. That is, because of the offset of the two opposing fluid directions **648** and **650,** half direct mixing and half indirect mixing occurs in the fluid mixing region.

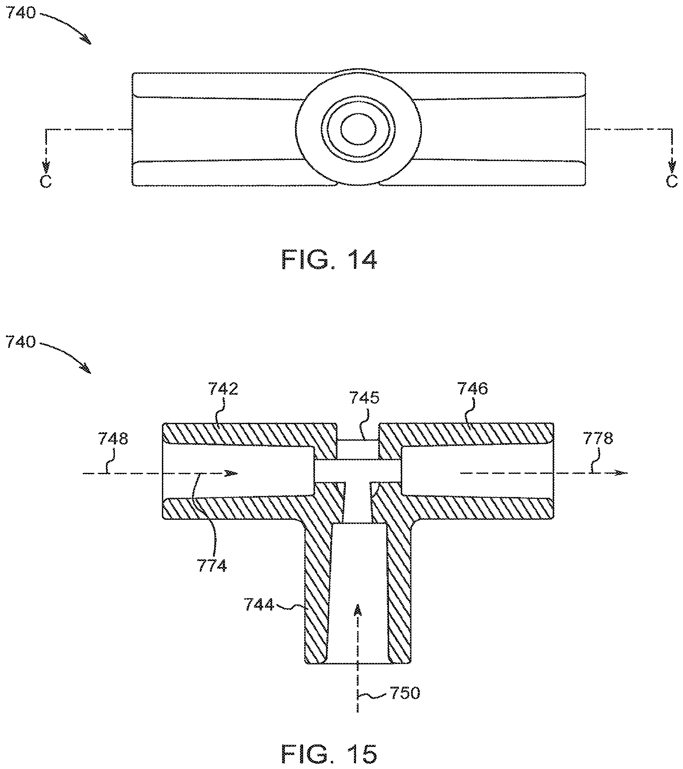

In yet another example not forming part of the invention of a fluid mixing device **740** of the present disclosure, as shown in **FIGS. 13-15****,** the first direction **748** is generally perpendicular to the second direction **750.** Moreover, the outlet port **746** of the fluid mixing device **740** may have an axis **778** generally parallel and coincidental to an axis **774** of the first fluid inlet **742.** In an alternative embodiment, fluid mixing device **740** (not shown) may have an axis **778** of an outlet port **746** generally parallel and coincidental to an axis of a second fluid inlet **744.** At least one notch **745** may be provided between two of the first fluid inlet **742,** the second fluid inlet **744,** and the outlet port **746.** The notch **745** may be provided to conserve material in a transition area between two of the first fluid inlet **742,** the second fluid inlet **744,** and the outlet port **746** to facilitate molding of the fluid mixing device **740.** According to these embodiments, the perpendicular collision of the flow paths of the first fluid and the second fluid in the fluid mixing device **740** may create turbulent mixing of the two fluids and limit and/or disrupt any laminar flow of one fluid relative to the other fluid.

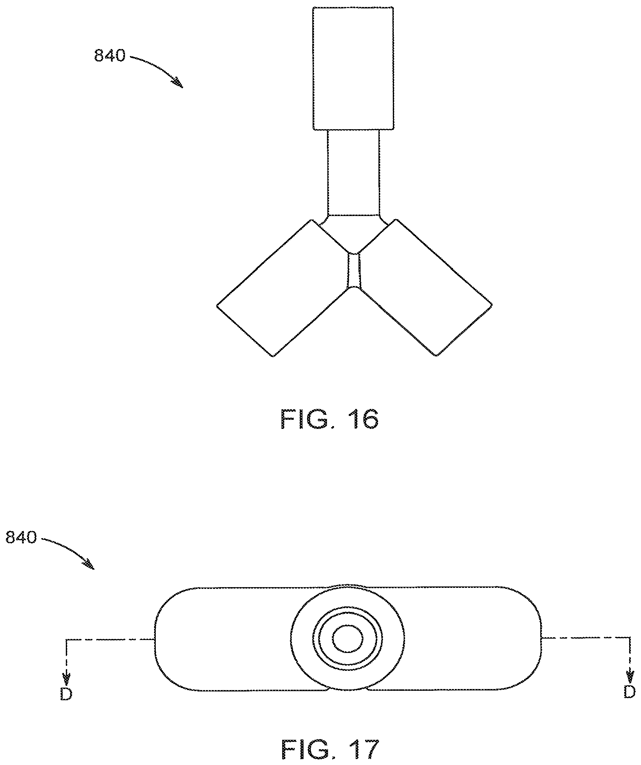

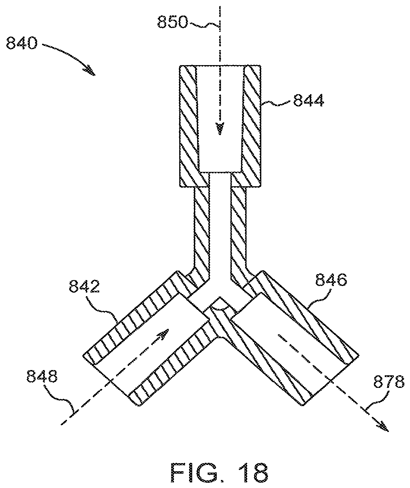

In yet another example not forming part of the invention of a fluid mixing device **840** of the present disclosure, as shown in **FIGS. 16-18****,** the first direction **848** may be at an angle of between 130° and 165° with respect to the second direction **850.** Additionally, the outlet port **846** of the fluid mixing device **840** may have an axis **878** at an angle less than 70° with respect to the first direction **848.** In an alternative embodiment, fluid mixing device **840** (not shown), the outlet port **846** may have an axis **878** at an angle less than 70° with respect to second direction **850.** According to these embodiments, the angled but substantially opposite flow of the flow paths of the first fluid and the second fluid in fluid mixing device **840** may create turbulent mixing of the two fluids and limit and/or disrupt any laminar flow of one fluid relative to the other fluid.

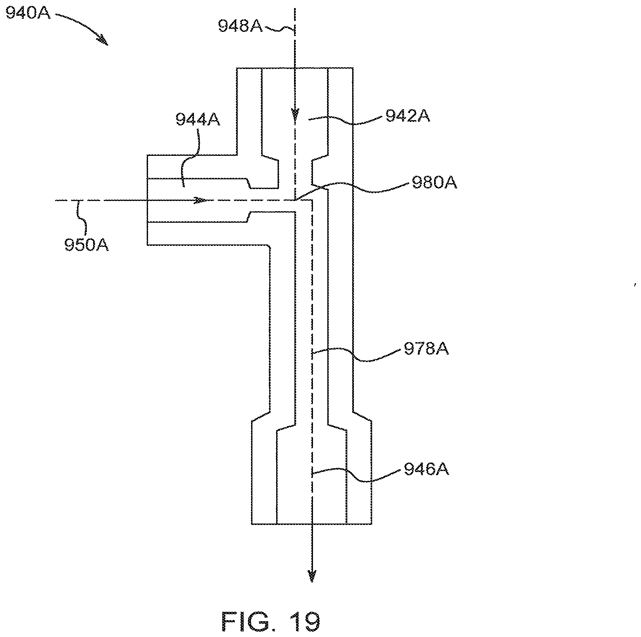

Other examples not forming part of the invention of fluid mixing devices **940A, 940B,** and **940C,** in accordance with various embodiments of the present disclosure, are shown in **FIGS. 19-21****.** According to these embodiments, the fluid mixing device **940A, 940B,** and **940C** has a T-shaped 90 degree connector design having one or more offset fluid paths to enhance mixing of the first fluid and the second fluid. Referring first to **FIG. 19****,** fluid mixing device **940A** includes a first fluid inlet **942A** and a second fluid inlet **944A** for a first fluid and a second fluid respectively, and a fluid outlet **946A.** As can be seen in **FIG. 19** the first fluid flow axis **948A** is offset from both the second fluid flow axis **950A** and the fluid outlet flow axis **978A.** Fluid mixing occurs at least in fluid mixing region **980A** where offset fluid flow lines of the first fluid along axis **948A** interact with fluid flow lines of the second fluid line along axis **950A** to create a turbulent mixing in fluid mixing region **980A,** which may be further enhanced by the offset of the outlet flow axis **978A** to the fluid outlet **946A.**

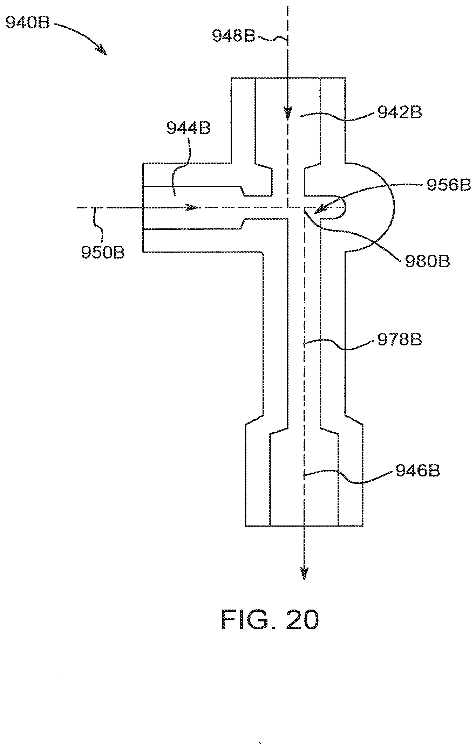

Referring to **FIG. 20****,** fluid mixing device **940B** includes a first fluid inlet **942B** and a second fluid inlet **944B** for a first fluid and a second fluid respectively, and a fluid outlet **946B.** The fluid mixing device **940B** also includes a turbulent fluid mixing chamber **956B** where further turbulent mixing may occur. As can be seen in **FIG. 20** the first fluid flow axis **948B** is offset from both the second fluid flow axis **950B** and the fluid outlet flow axis **978B.** Fluid mixing occurs at least in fluid mixing region **980B** where the fluid mixing chamber **956B** and the offset fluid flow lines of the first fluid along axis **948B** interact with fluid flow lines of the second fluid line along axis **950B** to create a turbulent mixing in fluid mixing region **980B,** which may be further enhanced by the offset of the outlet flow axis **978B** to fluid outlet **946B.**

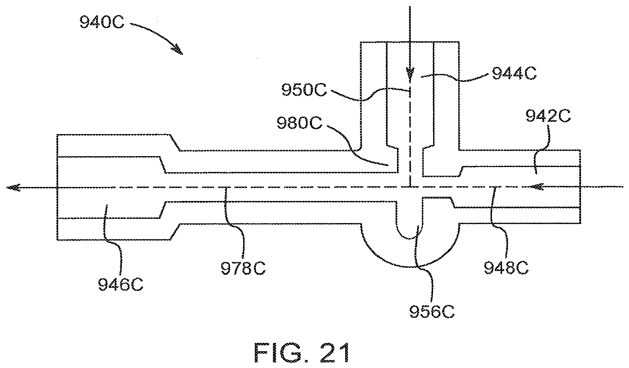

Referring to **FIG. 21****,** fluid mixing device **940C** includes a first fluid inlet **942C** and a second fluid inlet **944C** for a first fluid and a second fluid respectively, and a fluid outlet **946C.** The fluid mixing device **940C** also includes a turbulent fluid mixing chamber **956C** where further turbulent mixing may occur. As can be seen in **FIG. 21** the first fluid flow axis **948C** is offset from and the fluid outlet flow axis **978C,** particularly on the side of the flow path opposite the second fluid inlet **944C.** Fluid mixing occurs at least in fluid mixing region **980C** where the fluid mixing chamber **956C** and the fluid flow lines of the first fluid along axis **948C** interact with fluid flow lines of the second fluid line along axis **950C** to create a turbulent mixing in fluid mixing region **980C,** which may be further enhanced by the offset of the outlet flow axis **978C** to the fluid outlet **946C.**

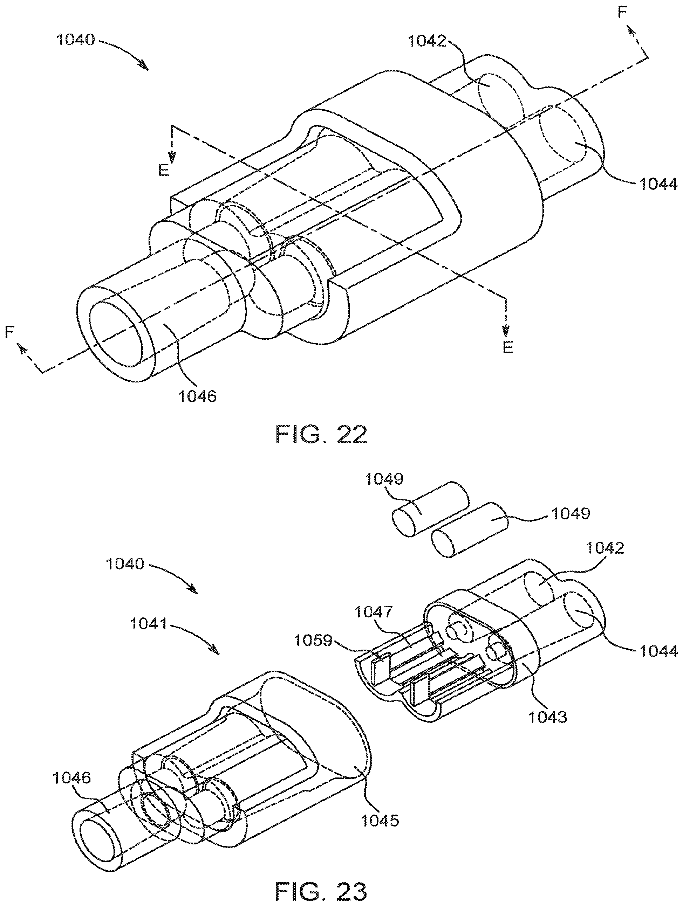

**FIG. 22** is a perspective view of a fluid mixing device **1040** according to some non-limiting embodiments of the present disclosure. The fluid mixing device **1040** may be used as part of a fluid delivery tube set, such as the fluid delivery tube set **202** shown in **FIG. 2****,** wherein the fluid mixing device **1040** is connected to a pair of fluid inlet lines and an outlet line. As shown in **FIG. 22****,** the fluid mixing device **1040** has a body defining first and second fluid inlets **1042** and **1044,** each of which is configured to conduct a corresponding one of the first and second injection fluids. The fluid mixing device **1040** further has an outlet port **1046** that is configured for delivering a mixture of the first and second injection fluids from the fluid mixing device **1040** to the patient or other downstream fluid path component.

With reference to **FIG. 23****,** which is an exploded perspective view of the fluid mixing device **1040** shown in **FIG. 22****,** the fluid mixing device **1040** has a body **1041**with a first portion **1043** and a second portion **1045.** In some embodiments, the first portion **1043** and the second portion **1045** may be manufactured separately and are connected together to form the body **1041** of the fluid mixing device **1040.** Desirably, the first portion **1043** and the second portion **1045** are connected together in a non-removable manner, such as by adhesive, welding (e.g., laser welding or ultrasonic welding), friction fit, solvent gluing, or other non-removable connection mechanism. In some embodiments, the first portion **1043** and the second portion **1045** may be removably connected together.

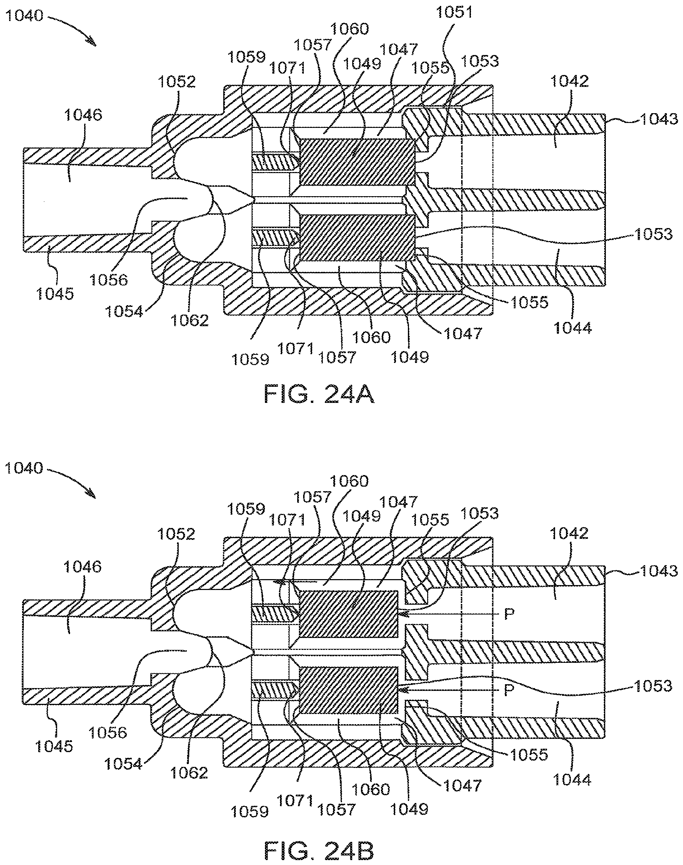

With continued reference to **FIG. 23****,** the first portion **1043** defines a portion of the first and second fluid inlets **1042** and **1044,** and has a receiving cavity **1047** for receiving a check valve **1049** in each of the first and second fluid inlets **1042** and **1044.** The second portion **1045** has a corresponding inner cavity **1051** (shown in **FIG. 24A**) that is configured to receive the first portion **1043,** including the check valves **1049.** A second part of the first and second fluid inlets **1042** and **1044** is defined by the inner cavity **1051** of the second portion **1045** (shown in **FIGS. 24A-24B**). Once the first portion **1043,** including the check valves **1049,** is inserted into the second portion **1045,** the first portion **1043** and the second portion **1045** may be joined together at one or more contact points between the first portion **1043** and the second portion **1045.**

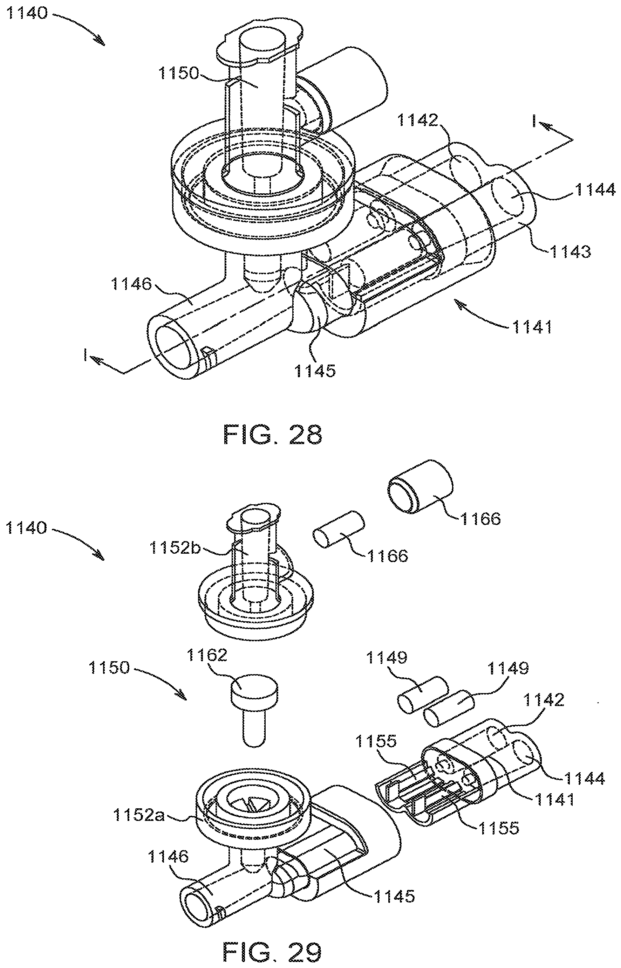

Each check valve **1049** may be configured to prevent backflow of the first and second injection fluids during injection procedures where fluid pressures in the respective first and second tubes delivering the first and second injection fluids to the fluid mixing device **1040** are not equal. The check valves **1049** may be made from a compressible material, such as an elastomeric polymer, that may be compressed under the pressurized flow of the fluid from an expanded state to a compressed state. The compressible material may be selected as appropriate to provide the appropriate stiffness so that the check valve opens at a selected fluid pressure. The check valves **1049** may also be used to isolate the fluid injector system from dampening a hemodynamic blood pressure signal, as discussed herein with reference to **FIGS. 28-30****.** In some embodiments, the check valves **1049** may be used to isolate contamination from patient to patient when the fluid mixing device **1040** is configured for multi-patient use. Furthermore, the check valves **1049** prevent "dribbling" of the first and second injection fluids to the outlet after the injection of first and second injection fluids ceases, such as due to release of built-up capacitance or "swelling" of the fluid injector components under pressure.

With reference to **FIGS. 24A-24B****,** which show a cross-sectional plan view of the fluid mixing device **1040** taken along line F - F shown in **FIG. 22****,** the check valves **1049** are shown positioned in the receiving cavity **1047** of each of the first and second fluid inlets **1042** and **1044** of the first portion **1043.** The receiving cavity **1047** for each valve **1049** is aligned with a direction of fluid flow through each of the first and second fluid inlets **1042** and **1044.** Each check valve **1049** has a proximal end **1053** that is configured to be in contact with a corresponding sealing face **1055** on the first and second fluid inlets **1042** and **1044** in the first portion **1043** when the check valve **1049** is in a closed position (**FIG. 24A**), and that is configured to be spaced apart from the sealing face **1055** on the first and second fluid inlets **1042** and **1044** in the first portion **1043** when the check valve **1049** is in an open position (**FIG. 24B**). Each check valve **1049** further has a distal end **1057** that is engaged with a stop element **1059** positioned within each of the first and second fluid inlets **1042** and **1044.** In some embodiments, each stop element **1059** may be a support structure that is connected to an inner sidewall of the respective first and second fluid inlet **1042, 1044** downstream of the check valve **1049** and is configured to prevent movement of the distal end **1057** of the check valve **1049,** thus allowing the check valve **1049** to compress when subject to a pressure force on the proximal end **1053.** In some embodiments, each stop element **1059** may have a pointed proximal end **1071** that is configured to reduce the contact area with the check valve **1049,** thereby allowing for a greater compression of the check valve **1049** between its proximal and distal ends **1053** and **1057** at a lower fluid pressure. For example, under pressure, the distal end **1057** may compress and mold around the pointed proximal end **1061** of the stop element **1059** allowing the outer circumference of the proximal end **1053** to more readily release from the sealing face **1055.** In this manner, the pointed stop element **1059** allows for decreased pressure drops by allowing easier opening during injections compared to stop elements with a flat supporting surface. In some embodiments, stop element **1059** is made from a silicone material.

During an injection procedure, the first and second injection fluids are urged under pressure through the first and second fluid inlets **1042** and **1044** such that the first and second fluids engage respective proximal ends **1053** of the check valves **1049.** Initially, the proximal ends **1053** engage the sealing face **1055** on the first portion **1043** (**FIG. 24A**) to block the passage of the first and second injection fluids past the check valve **1049.** As the fluid pressure builds, the force on the proximal end **1053** of the check valves **1049** increases. Due to the compressible nature of each check valve **1049,** the proximal end **1053** is urged in the distal direction, thereby creating a gap between the proximal end **1053** of the check valves **1049** and the sealing face **1055** on the first portion **1043.** As shown in **FIG. 24B****,** such a gap is formed only when sufficient fluid pressure P is imparted on the proximal end **1053,** such as, for example, during a typical injection procedure. The pressurized first and second injection fluids then travel around the respective check valves **1049** and through the fluid mixing device **1040,** as described herein. During the injection procedure, if the pressure of one of the first and second injection fluids is higher than the pressure of the other of the first and second injection fluids, the check valve **1049** in the fluid inlet with the lower pressure may close to prevent a backflow of the fluid in an upstream direction, for example due to the back pressure of the higher pressure fluid on the distal end **1055** of the lower pressure check valve **1049.** After the injection procedure is completed, the resilient nature of each check valve **1049** causes the check valve **1049** to expand axially such that the proximal end **1053** engages the sealing face **1055** on the first portion **1043** to prevent additional fluid from flowing past the check valve **1049.** In this manner, any excess fluid is prevented from flowing through the fluid mixing device **1040** after the completion of the injection procedure. Further, any backflow of one fluid into the other fluid path is prevented.

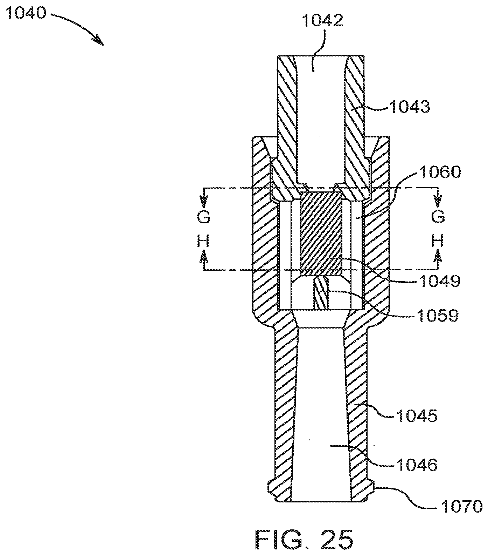

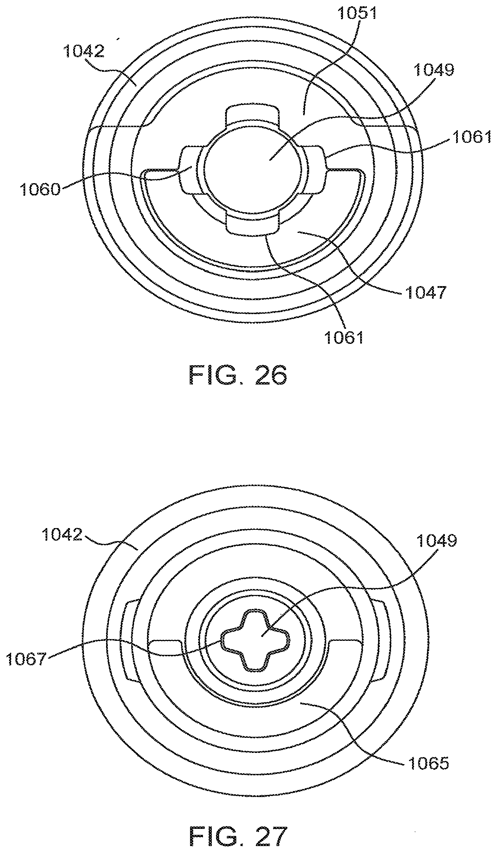

With reference to **FIG. 25****,** and with continued reference to **FIGS. 24A-24B****,** each check valve **1049** is dimensioned such that its outer diameter is slightly smaller than an inner diameter of a channel **1060** defined by the receiving cavity **1047** of the first portion **1043** (shown in **FIGS. 23A-24B**) and the corresponding inner cavity **1051** of the second portion **1045** of the body **1043** (shown in **FIG. 26**). In this manner, fluid may pass around the body of each check valve **1049** and through the channel **1060.** In some embodiments, the channel **1060** may have a non-circular cross-section and the check valve **1049** may have a circular cross-section. In this manner, the channel **1060** defines a flow path for the first and second injection fluids to flow around the respective check valves **1049,** when the check valve **1049** is in the open position.

In some embodiments, as shown in **FIG. 26****,** the channel **1060** may have a fluted cross-section with one or more flutes **1061.** In embodiments where the channel **1060** has a plurality of flutes **1061,** the flutes **1061** may be spaced apart from each other at equal or unequal spacing about a perimeter of the channel **1060.** The number of flutes **1061,** the radial depth, and/or the circumferential width of the flutes **1061** may be selected based on a desired flow rate of the first and second fluids through the channel **1061** when the respective check valves **1049** are in the open position.

Each check valve **1049** is desirably an elastomeric part that is at least partially compressible in a longitudinal direction when acted upon by fluid pressure. The check valve **1049** in the first fluid inlet **1042** may be the same or different compared to the check valve **1049** in the second fluid inlet **1044.** In some embodiments, the opening pressure of each check valve **1049** may be selected based on the characteristics of the fluid injector, and/or the characteristics of the first and second injection fluids, such as the fluid viscosity, and the temperature range, flow rate range, and the pressure range at which the first and second injections fluids will be injected.

With reference to **FIG. 27****,** an inlet opening **1065** surrounding the sealing face **1055** (shown in **FIG. 24A**) may have a shape that corresponds to the shape of the channel **1060** (shown in **FIG. 25**). The inlet opening **1065** may have a taper **1067** that tapers radially inward in a direction from the proximal end toward the distal end of the fluid mixing device **1040.** The cross-sectional shape of the inlet opening **1065** is chosen to achieve a low pressure drop and a lower opening pressure for the check valve **1049.**

With reference to **FIGS. 24A-24B****,** it will be appreciated that the fluid mixing device **1040** creates turbulent mixing of the first and second fluids similar to the fluid mixing device **240,** discussed herein. As shown in **FIGS. 24A-24B****,** the first and second fluid inlets **1042** and **1044** have corresponding first and second redirecting surfaces **1052** and **1054.** Moreover, the fluid mixing device **1040** further has a mixing chamber **1056** in fluid communication with the first and second fluid inlets **1042** and **1044** and an outlet port **1046** in fluid communication with the mixing chamber **1056.** The mixing chamber **1056** is configured to turbulently mix the first and second injection fluids together.

With continued reference to **FIGS. 24A-24B****,** the first and second redirecting surfaces **1052** and **1054** are configured to redirect a first fluid and a second fluid entering the first and second fluid inlets **1042** and **1044,** respectively, into the mixing chamber **1056,** where the first and second injection fluids can then be turbulently mixed. As discussed herein with reference to **FIG. 6****,** the first and second redirecting surfaces **1052** and **1054** are configured to redirect the first and second injection fluids in a corresponding first and second different directions that are different than the corresponding first and second directions in which the first and second injection fluids flow prior to contacting the first and second redirecting surfaces **1052** and **1054.** Due to this deflection, the first and second injection fluids enter the mixing chamber **1056** along the corresponding first and second different directions and contact a third redirecting surface **1062** at a proximal end of the mixing chamber **1056** to turbulently mix the first and second injections fluids together in the mixing chamber **1056.** After mixing, the mixture of the first and second injection fluids exits the fluid mixing device **1040** via the outlet port **1046** at a distal end of the fluid mixing device **1040.**

With reference to **FIG. 25****,** the outlet port **1046** may have a connection element **1070** configured for permitting removable connection of the outlet port **1046** with outlet tubing, such as the outlet line **220** shown in **FIG. 2****.** The connection element **1070** may be a male luer lock that is configured to removably connect with a corresponding female luer lock on the proximal end of the outlet line **220.** In some embodiments, the connection element **1070** may be a female luer lock that is configured to removably connect with a corresponding male luer lock on the proximal end of the outlet line **220.** In other embodiments, fluid path connectors such as described in International PCT Application Nos. PCT/US2021/018523 and PCT/US2016/063448. In this manner, the fluid mixing device **1040** can be removably connected to an outlet line **220** to thereby permit the use of the fluid mixing device **1040** with multiple patients, for example if one or more check valves are attached upstream of the connector on the outlet port **1046.**

In another embodiment of the present disclosure, as shown in **FIGS. 28-30****,** a fluid mixing device **1140** having a body **1141** defining first and second fluid inlets **1142** and **1144,** each of which is configured to conduct a corresponding one of the first and second injection fluids. The body of the fluid mixing device **1140** further includes an outlet port **1146** configured for delivering a mixture of the first and second injection fluids to outlet tubing (not shown). The body **1141** with a first portion **1143** and a second portion **1145** that are non-removably or removably connected together. A check valve **1149** is disposed in a channel **1155** of each of the first and second fluid inlets **1142** and **1144** (shown in **FIG. 29**) and is configured to be opened under pressure to permit a flow of the first and second injection fluids toward the outlet port **1146.** The structure and functionality of the fluid mixing device **1140** shown in **FIGS. 28-****30** is substantially identical to the structure and functionality of the fluid mixing device **1040** described herein with reference to **FIGS. 22-27****.** Accordingly, only the relative differences between the two embodiments will now be discussed.

With reference to **FIGS. 28-30****,** the outlet port **1146** may have a pressure isolation valve **1150** configured to allow for connecting a pressure transducer to the fluid path so that hemodynamic blood pressure signal readings may be obtained during fluid delivery. The pressure isolation valve **1150** isolates the high pressure fluid injector system from interfering with a low pressure measurement of a hemodynamic blood pressure signal.

The pressure isolation valve **1150** includes a housing **1152,** which may be a unitary structure or, preferably, a multi-piece structure as shown in **FIG. 29****.** For example, the housing **1152** is a two-piece housing including a first portion **1152a** and a second portion **1152b,** which are adapted to connect together to form the housing **1150.** The first and second portions **1152a, 1152b** are preferably formed for non-removable engagement with each other. Non-limiting examples of suitable pressure isolation valves are described in U.S. Patent Nos. 6,866,654; 7,611,503; 8,919,384; and 8,992,489.

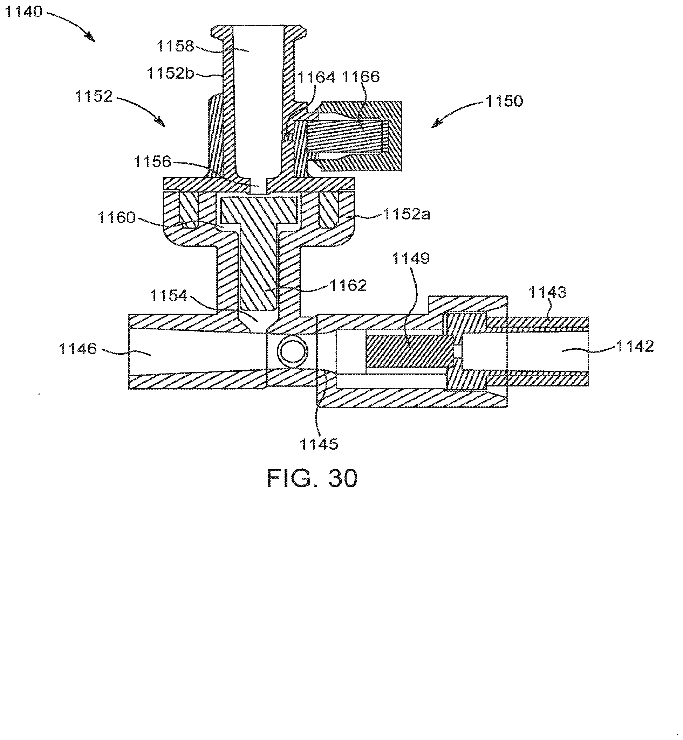

With reference to **FIG. 30****,** the first portion **1152a** of the housing **1152** defines a high pressure lumen **1154,** which forms a high pressure side of the pressure isolation valve **1150.** The high-pressure lumen **1154** is in fluid communication with the outlet port **1146.** The second portion **1152b** of the housing **1152** defines a low pressure lumen **1156,** which generally forms a low pressure side of the pressure isolation valve **1150.** The second portion **1152b** of the housing **1152** further includes a pressure isolation port **1158** to which a pressure transducer (not shown) may be connected. The structure forming pressure isolation port **1158** may terminate in a luer connector or other suitable medical connector for connecting a pressure transducer to the pressure isolation port **1158.**

The first and second portions **1152a, 1152b** of the housing **1152** may define an internal chamber **1160** generally in fluid communication with the high pressure lumen **1154** and the low pressure lumen **1156.** An internal valve member **1162** is located in the internal chamber **1160** and is biased to a normally open position, wherein the high pressure lumen **1154** is in fluid communication with the low pressure lumen **1156.** The valve member **1162** is generally further adapted to isolate the low pressure lumen **1156** once fluid pressure in the high pressure lumen **1154** reaches a preset pressure. The low pressure lumen **1156** further includes a flow initiating port **1164** having a flow initiating valve **1166** that is generally adapted to initiate a small flow around the valve member **1162** such that the valve member **1162** operates to a closed position substantially upon flow initiation.

While various embodiments of fluid mixing devices for mixing two injection fluids have been described herein, similar fluid mixing devices may exist with three or even four total fluid inlets, each having corresponding redirecting surfaces, where the fluid inlets are in fluid communication with a mixing chamber similar to as described herein. Such fluid mixing devices fall within the scope of the present disclosure.

## Claims

1. A fluid mixing device (240) for mixing a first injection fluid (F1) and a second injection fluid (F2), the fluid mixing device (240) comprising:
a first fluid inlet (242) configured to conduct the first injection fluid (F1) in a first direction (248) along a first axis (274) toward a first redirecting surface (252), the first fluid inlet (242) having a first concave-shaped redirecting surface (252);
a second fluid inlet (244) configured to conduct the second injection fluid (F2) in a second direction (250) along a second axis (276) different from the first axis (274) and toward a second concave-shaped redirecting surface (254), the second fluid inlet (244) having the second concave-shaped redirecting surface (254) , wherein the first axis (274) of the first direction (248) and the second axis (276) of the second direction (250) are substantially parallel;
a mixing chamber (256) in fluid communication with the first fluid inlet (242) and the second fluid inlet (244) and having a third concave-shaped redirecting surface (262); and
an outlet port (246) in fluid communication with the mixing chamber (256) and distal to the first fluid inlet (242) and the second fluid inlet (244),
wherein the first fluid inlet (242) and the second fluid inlet (244) have a first inlet port (264) and a second inlet port (266), respectively, wherein the first concave-shaped redirecting surface (252) and the second concave-shaped redirecting surface (254) are positioned distally relative to the first inlet port (242) and the second inlet port (244) and face the first inlet port (264) and the second inlet port (266), respectively, and wherein the third concave-shaped redirecting surface (262) is positioned proximally relative to the outlet port (246), the first concave-shaped redirecting surface (252), and the second concave-shaped redirecting surface (254) and wherein the concave-shaped surface of the third redirecting surface (262) faces the outlet port (246),
wherein the first concave-shaped redirecting surface (252) is configured to redirect the first injection fluid (F1) in a first different direction (258) from the first direction (248) to enter the mixing chamber (256) along the first different direction (258), and the second concave-shaped redirecting surface (254) is configured to redirect the second injection fluid (F2) in a second different direction (260) from the second direction (250) to enter the mixing chamber (256) along the second different direction (260), wherein the first different direction (258) and the second different direction (260) are angled toward each other with a 0° to 90° angle so that the first fluid (F1) and the second fluid (F2) directly impact each other in the mixing chamber (256) to turbulently mix, and wherein the first fluid (F1) and the second fluid (F2) in the mixing chamber (256) also contact the third concave-shaped redirecting surface (262) while turbulently mixing.

2. A fluid mixing device (240) of claim 1, wherein the third concave-shaped redirecting surface (262) redirects the turbulently mixed first fluid (F1) and second fluid (F2) toward the outlet port (246).

3. A fluid mixing device (240) of claim 1 or 2, wherein the mixing chamber (256) further comprises a first mixing chamber inlet (270), wherein the first mixing chamber inlet (270) is distal to the third concave-shaped redirecting surface (262), and wherein the first different direction (258) is at least partially angled towards the first mixing chamber inlet (270).

4. A fluid mixing device (240) of claim 3, wherein the mixing chamber (256) further comprises a second mixing chamber inlet (272), wherein the second mixing chamber inlet (272) is distal to the third concave-shaped redirecting surface (262), and wherein the second different direction (260) is at least partially angled towards the second mixing chamber inlet (256).

5. The fluid mixing device (240) of any of claims 1 to 4, wherein the outlet port (246) has an axis (278) parallel to an axis of the first direction (248) of the first fluid inlet (242) and an axis of the second direction (250) of the second fluid inlet (244).

6. The fluid mixing device (240) of any of claims 1 to 5, wherein the outlet port (246) further comprises a pressure isolation valve (1150) integrated therewith.

7. The fluid mixing device (240) of any of claims 1 to 6, further comprising a first check valve (1049) in the first fluid inlet (242) and a second check valve (1049) in the second fluid inlet (244).

8. The fluid mixing device (240) of claim 7, wherein the first check valve (1049) has a first end (1053) in engagement with a first inlet port (264) on the first fluid inlet (1042) and a second end (1057) in engagement with a first stop element (1059) proximal to the first redirecting surface (1052), wherein the second check valve (1049) has a first end (1053) in engagement with a second inlet port (266) on the second fluid inlet (1044) and a second end (1057) in engagement with a second stop element (1059) proximal to the second redirecting surface (1054), and wherein the first check valve (1049) and the second check valve (1049) are reversibly compressible between the first end (1053) and the second end (1057) in response to first fluid pressure of the first injection fluid (F1) flowing through the first inlet port (264) and a second fluid pressure of the second injection fluid (F2) flowing through the second fluid port (266), respectively.

9. The fluid mixing device (240) of claim 8, wherein the first stop element (1059) and the second stop element (1059) each have a pointed proximal end (1071).

10. A fluid delivery tube set (202) for delivering fluid from a fluid injector (100) to a patient, the fluid delivery tube set (202) comprising:
a first inlet tube (217a) configured to deliver a first injection fluid (F1);
a second inlet tube (217b) configured to deliver a second injection fluid (F2);
an outlet tube (220) configured to deliver a turbulently mixed mixture of the first injection fluid (F1) and the second injection fluid (F2) to the patient; and
the fluid mixing device (240) according to any of claims 1 to 9 configured to turbulently mix the mixture of the first injection fluid (F1) and the second injection fluid (F2).

## Patentansprüche

1. Eine Fluidmischvorrichtung (240) zum Mischen eines ersten Injektionsfluids (F1) und eines zweiten Injektionsfluids (F2), wobei die Fluidmischvorrichtung (240) umfasst:
einen ersten Fluideinlass (242), der dazu eingerichtet ist, das erste Injektionsfluid (F1) in einer ersten Richtung (248) entlang einer ersten Achse (274) zu einer ersten Umlenkfläche (252) zu leiten, wobei der erste Fluideinlass (242) eine erste konkav geformte Umlenkfläche (252) aufweist;
einen zweiten Fluideinlass (244), der dazu eingerichtet ist, das zweite Injektionsfluid (F2) in einer zweiten Richtung (250) entlang einer von der ersten Achse (274) verschiedenen zweiten Achse (276) zu einer zweiten konkav geformten Umlenkfläche (254) zu leiten, wobei der zweite Fluideinlass (244) die zweite konkav geformte Umlenkfläche (254) aufweist, wobei die erste Achse (274) der ersten Richtung (248) und die zweite Achse (276) der zweiten Richtung (250) im Wesentlichen parallel sind;
eine Mischkammer (256), die mit dem ersten Fluideinlass (242) und dem zweiten Fluideinlass (244) in Fluidverbindung steht und eine dritte konkav geformte Umlenkfläche (262) aufweist; und
eine Auslassöffnung (246), die mit der Mischkammer (256) in Fluidverbindung steht und distal zu dem ersten Fluideinlass (242) und dem zweiten Fluideinlass (244) angeordnet ist,
wobei der erste Fluideinlass (242) und der zweite Fluideinlass (244) jeweils eine erste Einlassöffnung (264) bzw. eine zweite Einlassöffnung (266) aufweisen, wobei die erste konkav geformte Umlenkfläche (252) und die zweite konkav geformte Umlenkfläche (254) distal relativ zu der ersten Einlassöffnung (242) bzw. der zweiten Einlassöffnung (244) angeordnet sind und der ersten Einlassöffnung (264) bzw. der zweiten Einlassöffnung (266) zugewandt sind, und wobei die dritte konkav geformte Umlenkfläche (262) proximal relativ zu der Auslassöffnung (246), der ersten konkav geformten Umlenkfläche (252) und der zweiten konkav geformten Umlenkfläche (254) angeordnet ist und wobei die konkav geformte Fläche der dritten Umlenkfläche (262) der Auslassöffnung (246) zugewandt ist,
wobei die erste konkav geformte Umlenkfläche (252) dazu eingerichtet ist, das erste Injektionsfluid (F1) aus der ersten Richtung (248) in eine erste unterschiedliche Richtung (258) umzulenken, um entlang der ersten unterschiedlichen Richtung (258) in die Mischkammer (256) einzutreten, und die zweite konkav geformte Umlenkfläche (254) dazu eingerichtet ist, das zweite Injektionsfluid (F2) aus der zweiten Richtung (250) in eine zweite unterschiedliche Richtung (260) umzulenken, um entlang der zweiten unterschiedlichen Richtung (260) in die Mischkammer (256) einzutreten, wobei die erste unterschiedliche Richtung (258) und die zweite unterschiedliche Richtung (260) mit einem Winkel von 0° bis 90° aufeinander zu ausgerichtet sind, sodass das erste Fluid (F1) und das zweite Fluid (F2) in der Mischkammer (256) direkt aufeinander auftreffen, um sich turbulent zu mischen, und wobei das erste Fluid (F1) und das zweite Fluid (F2) in der Mischkammer (256) während des turbulenten Mischens auch die dritte konkav geformte Umlenkfläche (262) berühren.

2. Fluidmischvorrichtung (240) nach Anspruch 1, wobei die dritte konkav geformte Umlenkfläche (262) das turbulent gemischte erste Fluid (F1) und das zweite Fluid (F2) in Richtung der Auslassöffnung (246) umlenkt.

3. Fluidmischvorrichtung (240) nach Anspruch 1 oder 2, wobei die Mischkammer (256) ferner einen ersten Mischkammereinlass (270) umfasst, wobei der erste Mischkammereinlass (270) distal zu der dritten konkav geformten Umlenkfläche (262) angeordnet ist, und wobei die erste unterschiedliche Richtung (258) zumindest teilweise zu dem ersten Mischkammereinlass (270) hin abgewinkelt ist.

4. Fluidmischvorrichtung (240) nach Anspruch 3, wobei die Mischkammer (256) ferner einen zweiten Mischkammereinlass (272) umfasst, wobei der zweite Mischkammereinlass (272) distal zu der dritten konkav geformten Umlenkfläche (262) angeordnet ist, und wobei die zweite unterschiedliche Richtung (260) zumindest teilweise zu dem zweiten Mischkammereinlass (256) hin abgewinkelt ist.

5. Fluidmischvorrichtung (240) nach einem der Ansprüche 1 bis 4, wobei die Auslassöffnung (246) eine Achse (278) aufweist, die parallel zu einer Achse der ersten Richtung (248) des ersten Fluideinlasses (242) und zu einer Achse der zweiten Richtung (250) des zweiten Fluideinlasses (244) ist.

6. Fluidmischvorrichtung (240) nach einem der Ansprüche 1 bis 5, wobei die Auslassöffnung (246) ferner ein darin integriertes Druckisolationsventil (1150) umfasst.

7. Fluidmischvorrichtung (240) nach einem der Ansprüche 1 bis 6, ferner umfassend ein erstes Rückschlagventil (1049) in dem ersten Fluideinlass (242) und ein zweites Rückschlagventil (1049) in dem zweiten Fluideinlass (244).

8. Fluidmischvorrichtung (240) nach Anspruch 7, wobei das erste Rückschlagventil (1049) ein erstes Ende (1053) in Eingriff mit einer ersten Einlassöffnung (264) an dem ersten Fluideinlass (1042) und ein zweites Ende (1057) in Eingriff mit einem ersten Anschlagelement (1059) proximal zu der ersten Umlenkfläche (1052) aufweist, wobei das zweite Rückschlagventil (1049) ein erstes Ende (1053) in Eingriff mit einer zweiten Einlassöffnung (266) an dem zweiten Fluideinlass (1044) und ein zweites Ende (1057) in Eingriff mit einem zweiten Anschlagelement (1059) proximal zu der zweiten Umlenkfläche (1054) aufweist, und wobei das erste Rückschlagventil (1049) und das zweite Rückschlagventil (1049) als Reaktion auf einen ersten Fluiddruck des durch die erste Einlassöffnung (264) strömenden ersten Injektionsfluids (F1) bzw. einen zweiten Fluiddruck des durch die zweite Fluidöffnung (266) strömenden zweiten Injektionsfluids (F2) zwischen dem ersten Ende (1053) und dem zweiten Ende (1057) reversibel komprimierbar sind.

9. Fluidmischvorrichtung (240) nach Anspruch 8, wobei das erste Anschlagelement (1059) und das zweite Anschlagelement (1059) jeweils ein zugespitztes proximales Ende (1071) aufweisen.

10. Ein Fluidzufuhrschlauchsatz (202) zum Zuführen von Fluid von einem Fluidinjektor (100) zu einem Patienten, wobei der Fluidzufuhrschlauchsatz (202) umfasst:
einen ersten Einlassschlauch (217a), der dazu eingerichtet ist, ein erstes Injektionsfluid (F1) zuzuführen;
einen zweiten Einlassschlauch (217b), der dazu eingerichtet ist, ein zweites Injektionsfluid (F2) zuzuführen;
einen Auslassschlauch (220), der dazu eingerichtet ist, ein turbulent gemischtes Gemisch des ersten Injektionsfluids (F1) und des zweiten Injektionsfluids (F2) dem Patienten zuzuführen; und
die Fluidmischvorrichtung (240) nach einem der Ansprüche 1 bis 9, die dazu eingerichtet ist, das Gemisch des ersten Injektionsfluids (F1) und des zweiten Injektionsfluids (F2) turbulent zu mischen.

## Revendications

1. Un dispositif de mélange de fluides (240) destiné à mélanger un premier fluide d'injection (F1) et un second fluide d'injection (F2), le dispositif de mélange de fluides (240) comprenant:
une première entrée de fluide (242) configurée pour acheminer le premier fluide d'injection (F1) dans une première direction (248) le long d'un premier axe (274) vers une première surface de redirection (252), la première entrée de fluide (242) ayant une première surface de redirection concave (252);
une seconde entrée de fluide (244) configurée pour acheminer le second fluide d'injection (F2) dans une seconde direction (250) le long d'un second axe (276) différent du premier axe (274) et vers une seconde surface de redirection concave (254), la seconde entrée de fluide (244) ayant la seconde surface de redirection concave (254), dans lequel le premier axe (274) de la première direction (248) et le second axe (276) de la seconde direction (250) sont sensiblement parallèles;
une chambre de mélange (256) en communication fluidique avec la première entrée de fluide (242) et la seconde entrée de fluide (244) et ayant une troisième surface de redirection concave (262); et
un orifice de sortie (246) en communication fluidique avec la chambre de mélange (256) et distal par rapport à la première entrée de fluide (242) et à la seconde entrée de fluide (244),
dans lequel la première entrée de fluide (242) et la seconde entrée de fluide (244) ont respectivement un premier orifice d'entrée (264) et un second orifice d'entrée (266), dans lequel la première surface de redirection concave (252) et la seconde surface de redirection concave (254) sont positionnées distalement par rapport au premier orifice d'entrée (242) et au second orifice d'entrée (244) et font face respectivement au premier orifice d'entrée (264) et au second orifice d'entrée (266), et dans lequel la troisième surface de redirection concave (262) est positionnée proximalement par rapport à l'orifice de sortie (246), à la première surface de redirection concave (252) et à la seconde surface de redirection concave (254), et dans lequel la surface concave de la troisième surface de redirection (262) fait face à l'orifice de sortie (246),
dans lequel la première surface de redirection concave (252) est configurée pour rediriger le premier fluide d'injection (F1) dans une première direction différente (258) de la première direction (248) afin d'entrer dans la chambre de mélange (256) le long de la première direction différente (258), et la seconde surface de redirection concave (254) est configurée pour rediriger le second fluide d'injection (F2) dans une seconde direction différente (260) de la seconde direction (250) afin d'entrer dans la chambre de mélange (256) le long de la seconde direction différente (260), dans lequel la première direction différente (258) et la seconde direction différente (260) sont inclinées l'une vers l'autre selon un angle de 0° à 90°, de sorte que le premier fluide (F1) et le second fluide (F2) se heurtent directement l'un l'autre dans la chambre de mélange (256) en se mélangeant de manière turbulente, et dans lequel le premier fluide (F1) et le second fluide (F2) dans la chambre de mélange (256) entrent également en contact avec la troisième surface de redirection concave (262) tout en se mélangeant de manière turbulente.

2. Un dispositif de mélange de fluides (240) selon la revendication 1, dans lequel la troisième surface de redirection concave (262) redirige le premier fluide (F1) et le second fluide (F2) mélangés de manière turbulente vers l'orifice de sortie (246).

3. Un dispositif de mélange de fluides (240) selon la revendication 1 ou 2, dans lequel la chambre de mélange (256) comprend en outre une première entrée de chambre de mélange (270), dans lequel la première entrée de chambre de mélange (270) est distale par rapport à la troisième surface de redirection concave (262), et dans lequel la première direction différente (258) est au moins partiellement inclinée vers la première entrée de chambre de mélange (270).

4. Un dispositif de mélange de fluides (240) selon la revendication 3, dans lequel la chambre de mélange (256) comprend en outre une seconde entrée de chambre de mélange (272), dans lequel la seconde entrée de chambre de mélange (272) est distale par rapport à la troisième surface de redirection concave (262), et dans lequel la seconde direction différente (260) est au moins partiellement inclinée vers la seconde entrée de chambre de mélange (256).

5. Le dispositif de mélange de fluides (240) selon l'une quelconque des revendications 1 à 4, dans lequel l'orifice de sortie (246) a un axe (278) parallèle à un axe de la première direction (248) de la première entrée de fluide (242) et à un axe de la seconde direction (250) de la seconde entrée de fluide (244).

6. Le dispositif de mélange de fluides (240) selon l'une quelconque des revendications 1 à 5, dans lequel l'orifice de sortie (246) comprend en outre une soupape d'isolation de pression (1150) intégrée à celui-ci.

7. Le dispositif de mélange de fluides (240) selon l'une quelconque des revendications 1 à 6, comprenant en outre une première soupape de retenue (1049) dans la première entrée de fluide (242) et une seconde soupape de retenue (1049) dans la seconde entrée de fluide (244).

8. Le dispositif de mélange de fluides (240) selon la revendication 7, dans lequel la première soupape de retenue (1049) a une première extrémité (1053) en prise avec un premier orifice d'entrée (264) sur la première entrée de fluide (1042) et une seconde extrémité (1057) en prise avec un premier élément d'arrêt (1059) proximal à la première surface de redirection (1052), dans lequel la seconde soupape de retenue (1049) a une première extrémité (1053) en prise avec un second orifice d'entrée (266) sur la seconde entrée de fluide (1044) et une seconde extrémité (1057) en prise avec un second élément d'arrêt (1059) proximal à la seconde surface de redirection (1054), et dans lequel la première soupape de retenue (1049) et la seconde soupape de retenue (1049) sont réversiblement compressibles entre la première extrémité (1053) et la seconde extrémité (1057) en réponse à une première pression de fluide du premier fluide d'injection (F1) s'écoulant à travers le premier orifice d'entrée (264) et à une seconde pression de fluide du second fluide d'injection (F2) s'écoulant à travers le second orifice de fluide (266), respectivement.

9. Le dispositif de mélange de fluides (240) selon la revendication 8, dans lequel le premier élément d'arrêt (1059) et le second élément d'arrêt (1059) ont chacun une extrémité proximale pointue (1071).

10. Un ensemble de tubes d'administration de fluide (202) destiné à administrer un fluide d'un injecteur de fluide (100) à un patient, l'ensemble de tubes d'administration de fluide (202) comprenant:
un premier tube d'entrée (217a) configuré pour administrer un premier fluide d'injection (F1);
un second tube d'entrée (217b) configuré pour administrer un second fluide d'injection (F2);
un tube de sortie (220) configuré pour administrer au patient un mélange, mélangé de manière turbulente, du premier fluide d'injection (F1) et du second fluide d'injection (F2); et
le dispositif de mélange de fluides (240) selon l'une quelconque des revendications 1 à 9, configuré pour mélanger de manière turbulente le mélange du premier fluide d'injection (F1) et du second fluide d'injection (F2).
